# FASCICULE DE BREVET EUROPEEN

(11) **EP 0 562 906 B1**
(45) Date de publication et mention de la délivrance du brevet: **12.06.1996**
(21) Numéro de dépôt: 93400645.3
(22) Date de dépôt: 12.03.1993
(51) Int. Cl.: C07C 5/333, C07C 11/02

(54) **Procédé continu de déshydrogénation d'hydrocarbures paraffiniques en hydrocarbures oléfiniques**
Kontinuierliches Verfahren zur Dehydrierung von paraffinischen Kohlenwasserstoffen zu olefinischen Kohlenwasserstoffen
Continuous process for the dehydration of paraffinic hydro-carbons to olefinic hydrocarbons

(30) Priorité: 26.03.1992 FR 9203790
(43) Date de publication de la demande: 29.09.1993
(73) Titulaire: INSTITUT FRANCAIS DU PETROLE, F-92502 Rueil-Malmaison (FR)
(72) Inventeur: Boitiaux, Jean-Paul, F-78300 Poissy (FR); De Bonneville, Jean, F-78380 Bougival (FR); Burzynski, Jean-Pierre, F-69110 Sainte-Foy-les-Lyon (FR); Léger, Gérard, F-69300 Caluire (FR); Le Peltier, Fabienne, F-92500 Rueil Malmaison (FR); Martino, Germain, F-78300 Poissy (FR)

(56) Documents cités:
- EP-A- 0 212 850
- FR-A- 2 310 399
- US-A- 3 531 543
- US-A- 3 978 150

## Description

L'invention concerne un procédé continu de déshydrogénation d'hydrocarbures paraffiniques, le plus souvent gazeux à température ambiante, comportant au moins 2 atomes de carbone dans leur molécule en hydrocarbures oléfiniques correspondants.

La présente invention concerne plus particulièrement la synthèse d'isobutène par déshydrogénation d'isobutane. L'isobutène est utilisé notamment pour la préparation du MTBE (méthyl tertio-butyl éther) en vue de l'amélioration de l'indice d'octane des essences.

Le procédé de la présente invention comprend des moyens d'injection de soufre dans la charge à traiter et des moyens de strippage du soufre retenu sur le catalyseur ; ces conditions permettent d'améliorer la stabilité du catalyseur ainsi que sa durée de vie.

La valorisation des coupes hydrocarbonées saturées en particulier des hydrocarbures aliphatiques saturés à bas point d'ébullition tels que l'éthane, le propane, le butane, l'isobutane, le pentane et l'isopentane que l'on peut notamment récupérer après extraction des insaturés des coupes C3, C4, et C5 de vapocraquage ou de craquage catalytique et également à partir des gaz de pétrole liquéfié souvent dénommés LPG ou à partir des gaz de champ, justifie l'intérêt que l'on peut porter à la mise en oeuvre de procédés de déshydrogénation de ces hydrocarbures qui soient performants, sélectifs et économiques tout en contribuant également à la formation d'hydrogène, produit à tendance déficitaire dans les schémas de raffinage actuels.

La réaction de production d'hydrocarbures aliphatiques oléfiniques, souvent dénommés alcènes par le chimiste, à partir d'hydrocarbures aliphatiques saturés, souvent dénommés alcanes par le chimiste, est connue depuis très longtemps. Cette réaction a été décrite, notamment dans les brevets US-A-3 126 426, US-A-3 531 543, US-A-3 978 150, US-A-4 381 417, US-A-4 381 418 et US-A-4 704 497. Elle peut mettre en oeuvre un catalyseur métallique supporté à base de platine, un oxyde de chrome sur alumine ou bien un catalyseur zéolithique cristallin à base de silice et d'alumine de type MFI.

Un des problèmes à résoudre est relatif à la tenue au cokage des matériaux du réacteur, des lignes et des échangeurs inter-réacteurs ; en effet dans les conditions de la réaction (température élevée et présence d'oléfines) les matériaux usuels catalysent les réactions de déshydrogénation et conduisent à un cokage relativement rapide de la surface métallique.

Ce point est particulièrement critique dans le cas ou l'on opère en système régénératif, c'est-à-dire en continu, car dans ce cas il n'est pas possible d'interrompre le fonctionnement des réacteurs pour un décokage mécanique ou pour un brûlage du coke sans dénaturer complètement le procédé.

De façon surprenante il a été découvert que l'ajout de soufre à la charge permet de passiver les surfaces métalliques sans porter un préjudice sévère au procédé et en particulier à la conversion et à la sélectivité en oléfines.

Globalement, la réaction est endothermique, la vitesse de réaction est sensible aux variations de température et les différentes réactions s'accompagnent d'un dépôt de coke sur le catalyseur et dans une certaine mesure d'un frittage des particules métalliques contenues dans le catalyseur, ce qui le désactive très rapidement et réduit sa durée de cycle.

Un autre problème est la forte désactivation du catalyseur aux températures élevées de réaction. On a constaté que l'ajout de soufre à la charge ou bien la présulfuration du catalyseur peut être un moyen d'améliorer sa stabilité.

La régénération du catalyseur peut se faire en système cyclique ou continu (régénératif).

La demande de brevet européen EP-A-0 212 850 décrit la déshydrogénation en présence d'un catalyseur comprenant un silicalite, un métal du groupe du platine et ne comprenant pas d'alcalin ou d'alcalino-terreux. Dans ladite demande, il est décrit l'ajout de soufre par présulfuration du catalyseur ou par passage d'un produit soufré dans la charge.

L'objet de l'invention concerne un procédé continu de déshydrogénation d'hydrocarbures paraffiniques, ayant au moins deux atomes de carbone dans leur molécule, en hydrocarbures oléfiniques, en présence d'un catalyseur, dans lequel on fait circuler une charge contenant lesdits hydrocarbures paraffiniques à travers au moins deux zones de réaction disposées en série, chacune de ces zones de réaction étant de type à lit mobile, la charge circulant successivement à travers chaque zone de réaction et le catalyseur circulant également successivement à travers chaque zone de réaction en s'écoulant en continu ou de façon périodique depuis une première extrémité de ladite zone vers son extrémité opposée, le catalyseur soutiré à partir de l'extrémité de chaque zone de réaction (sauf de la dernière) étant transporté dans un courant de gaz à la première extrémité de la zone de réaction suivante, le catalyseur soutiré de la dernière zone de réaction traversée par la charge étant ensuite envoyé dans une zone de régénération à la sortie de laquelle le catalyseur régénéré est réintroduit de façon continue ou périodique à proximité de la première extrémité de la première zone de réaction, ledit procédé comprenant une injection de soufre et/ou d'au moins un composé soufré préalablement ou simultanément à l'introduction de la charge dans la première zone de réaction et étant caractérisé en ce que le catalyseur soutiré de la dernière zone de réaction traversée par la charge est envoyé de façon continue ou périodique dans une zone de strippage dans laquelle il est débarrassé du soufre qu'il renferme, par strippage à l'aide d'un gaz ou d'un mélange de gaz, avant d'être envoyé de façon continue ou périodique dans la zone de régénération.

Le gaz de transport entre les réacteurs peut être de l'hydrogène, de l'azote ou un mélange de ces gaz.

Plus particulièrement la présente invention concerne la production d'hydrocarbures oléfiniques à partir d'une charge comprenant des hydrocarbures aliphatiques saturés ayant le plus souvent de 2 à 5 atomes de carbone et de préférence de 3 à 5 atomes de carbone dans leur molécule. Ces hydrocarbures peuvent être du propane, du n-butane, du n-pentane, les isomères du butane et du pentane et leurs mélanges.

Le procédé comprend des moyens d'injection de soufre dans la charge à traiter et des moyens de strippage du soufre retenu sur le catalyseur, car cela permet de manière surprenante d'améliorer la stabilité du catalyseur ainsi que sa durée de vie sans influer notablement sur la sélectivité en oléfines.

Dans le procédé de l'invention la charge peut être ou non prémélangée avec un diluant choisi par exemple dans le groupe formé par l'hydrogène, la vapeur d'eau et le méthane.

La charge circule à travers au moins deux zones catalytiques élémentaires placées en série, le plus souvent verticales, avec chauffage intermédiaire entre chaque zone ; ces zones catalytiques sont le plus souvent constituées chacune d'un lit mobile à écoulement radial. Ces zones sont par exemple identiques à celles décrites dans le brevet US-A-4 277 444. La pression dans les zones catalytiques est maintenue aussi basse que possible, compatible avec les pertes de charge des lignes, des réacteurs et des échangeurs de façon à tirer avantage de l'équilibre thermodynamique. Le soufre peut être injecté dans la charge sous forme de soufre et/ou sous forme d'au moins un composé soufré.

Le plus souvent on introduit dans la charge un composé organique soufré. Les composés organiques soufrés que l'on injecte habituellement sont choisi dans le groupe formé par les disulfures et les polysulfures organiques, et de préférence ceux de formules R¹-(S)ₙ-R² dans laquelle R¹ et R², identiques ou différents, représentent chacun un atome d'hydrogène ou un radical hydrocarboné et n est un nombre compris entre 2 et 20 (bornes incluses) et de préférence entre 2 et 8 (bornes incluses).

Parmi les radicaux hydrocarbonés on peut citer, à titre d'exemple, les radicaux aliphatiques saturés ou insaturés, linéaires ou ramifiés, les radicaux cycloaliphatiques et les radicaux aryles. Dans la formule ci-avant, R¹ et R², identiques ou différents, représentent, par exemple, indépendamment l'un de l'autre, un atome d'hydrogène, un radical alkyle linéaire ou ramifié, un radical aryle, un radical aryle-alkyle ou un radical cycloalkyle.

On utilise avantageusement des polysulfures de formule ci-avant dans laquelle l'un au moins des radicaux R¹ et R² représente un radical hydrocarboné et de préférence ceux dans lesquels R¹ et R², identiques ou différents, représentent chacun un radical hydrocarboné tel que par exemple un radical alkyle linéaire ou ramifié ou un radical aryle. Les polysulfures organiques particulièrement préférés sont ceux dans lesquels R¹ et R², identiques ou différents, représentent chacun un radical alkyle ; ils sont dénommés ci-après dialkylpolysulfures.

A titre d'exemple de polysulfures organiques on peut citer les disulfures organiques et plus particulièrement les dialkyldisulfures.

Les polysulfures organiques employés dans la présente invention ont habituellement de 2 à 72 atomes de carbone et de préférence de 2 à 48 atomes de carbone dans leur molécule.

Les dialkylpolysulfures employés ont avantageusement de 2 à 24 atomes de carbone dans leur molécule et les polysulfures organiques ayant la formule ci-avant dans laquelle R¹ et R² représentent un radical aryle, aralkyle ou cycloalkyle ont avantageusement de 6 à 48 atomes de carbone et de préférence de 10 à 32 atomes de carbone dans leur molécule.

A titre d'exemple de composés spécifiques de polysulfures organiques, on peut citer le diméthyldisulfure (DMDS), le diéthyldisulfure (DEDS), les dipropyldisulfures, les dibutyldisulfures et en particulier le ditertiobutyldisulfure (DTBDS), le diphényldisulfure (DPDS), le ditertiododécylpolysulfure (n=5) commercialisé par exemple par ELF AQUITAINE sous le nom TPS 32, notamment parce qu'il contient environ 32 % en poids de soufre, et le ditertiononylpolysulfure (n=5) commercialisé par exemple par ELF AQUITAINE sous le nom TPS 37.

La quantité de polysulfure, employée dans la présente invention, exprimée en poids de soufre par rapport à la charge est habituellement de 1 à 1000 ppm, et le plus souvent de 1 à 200 ppm. Cette quantité est de préférence d'environ 5 à 100 ppm.

Dans chaque zone verticale le catalyseur est en mouvement lent et est soutiré de manière continue ou bien périodiquement de la dernière zone catalytique pour être envoyé dans une zone de régénération à la sortie de laquelle le catalyseur régénéré est renvoyé en tête de la première zone catalytique afin de maintenir une activité sensiblement constante dans chacune des zones.

On adopte pour le soutirage du catalyseur issu de la dernière zone catalytique une fréquence et un taux convenables. Par exemple le catalyseur peut être soutiré soit en continu, soit périodiquement par exemple, avec une fréquence de 1/10 à 10 jours, en ne soutirant à la fois qu'une fraction par exemple 0,5 à 15 % de la quantité totale de catalyseur. Il est également possible de soutirer ce catalyseur avec une fréquence beaucoup plus rapide de l'ordre de la minute ou de la seconde ; la quantité soutirée est alors réduite en proportion.

Le catalyseur usé ou partiellement usé, provenant de la dernière zone catalytique est alors dirigé vers une zone de strippage où il est débarrassé du soufre accumulé dans les zones de réaction. Cette opération est réalisée en injectant un fluide de strippage à une température comprise entre 400 °C et 700 °C de préférence 500 °C et 600 °C, à une pression comprise entre 1 et 4 bar de préférence 1,5 et 2,5 bar et à une VVH comprise entre 100 h⁻¹ et 4 000 h⁻¹ de préférence 700 et 2 500 h⁻¹.

Le fluide de strippage est habituellement choisi dans le groupe formé par l'hydrogène sensiblement pur, l'hydrogène renfermant de 5 à 10 000 ppm en poids d'eau et/ou de 5 à 5 000 ppm en poids d'un halogène, l'azote sensiblement pur, l'azote renfermant de 5 à 10 000 ppm en poids d'eau et/ou de 5 à 5 000 ppm en poids d'un halogène, les mélanges d'azote sensiblement pur et d'hydrogène sensiblement pur, et les mélanges d'azote et d'hydrogène renfermant de 5 à 10 000 ppm en poids d'eau et/ou de 5 à 5 000 ppm en poids d'un halogène. Cet halogène est le plus souvent du chlore.

Dans une première variante, le fluide de strippage peut être de préférence de l'hydrogène de recyclage éventuellement purifié du soufre issu de la charge ou du catalyseur si celui-ci a été présulfuré. Le catalyseur dans la zone de strippage peut être disposé en lit axial ou bien radial.

Le strippage peut être effectué en continu ou bien de manière périodique .

Le catalyseur débarrassé du soufre peut alors être remonté par un lift vers un ballon "accumulateur décanteur" situé au dessus du régénérateur. Le fluide du lift, utilisé pour convoyer le catalyseur peut être, de préférence, l'hydrogène de recyclage. Dans le ballon "accumulateur décanteur" l'hydrogène est séparé du catalyseur et dirigé vers un filtre pour éliminer les particules fines de catalyseur, et de là envoyé vers un compresseur en vue de son recyclage, par exemple vers l'espace de réaction, vers les lifts ou vers la zone de strippage.

Selon une variante du procédé la zone de strippage peut être placée après le ballon "accumulateur décanteur" et avant le régénérateur proprement dit.

La régénération du catalyseur peut être effectuée en mode discontinu ou en mode continu par tout moyen connu. La zone de régénération doit être maintenue, au cours de la phase de régénération, exempte de toute trace d'hydrogène. En mode discontinu le catalyseur usé ou partiellement usé s'accumule dans le ballon "accumulateur décanteur" avant d'alimenter le régénérateur situé généralement en dessous de ce ballon. A intervalles réguliers le régénérateur est mis en équilibre de pression par exemple sous atmosphère d'hydrogène avec le ballon "accumulateur décanteur"; il est ensuite rempli de catalyseur provenant à travers un système de vannes du ballon "accumulateur décanteur" puis isolé du reste du système. Le régénérateur est alors purgé à l'azote pour éliminer l'hydrogène et les hydrocarbures et l'on effectue la régénération du catalyseur. Le régénérateur, ou un ballon accumulateur dans lequel on a transféré le catalyseur régénéré, est ensuite purgé à l'azote, puis mis sous hydrogène en équilibre de pression avec la zone catalytique dans laquelle le catalyseur va être injecté.

La régénération proprement dite, en particulier dans le cas d'un catalyseur supporté sur alumine, est habituellement effectuée de la manière suivante :
- un premier traitement correspond à la combustion des dépôts de coke. Cette opération est réalisée par l'injection d'air dans un mélange inerte (composé, par exemple d'azote et de gaz carbonique), ce mélange inerte servant de diluant thermique. La teneur en oxygène dans le gaz de régénération injecté en tête du régénérateur est de préférence comprise entre 0.01 et 2 % en volume. L'air injecté est consommé par la combustion des dépôts de coke et la fin de la combustion se détecte aisément par l'augmentation de la teneur en oxygène dans le gaz sortant du régénérateur et également par la disparition du front de flamme (plan horizontal où se produit la combustion) qui se propage de haut en bas du lit catalytique. La combustion est réalisée à une température moyenne comprise de préférence entre 350 et 550 °C et sous une pression comprise, par exemple, entre 1 et 15 bar;
- un deuxième traitement correspond à l'oxychloration du catalyseur ; à cette fin on augmente la teneur en oxygène du gaz de régénération admis dans le régénérateur de préférence d'au moins 10 % par rapport à la valeur dans la première étape. La teneur en oxygène est le plus souvent comprise, au cours de cette étape d'oxychloration, entre 1 et 8 % volume. Au cours de cette étape on introduit simultanément un halogène et/ou un composé halogéné. On utilise de préférence du chlore et/ou un composé chloré. On emploie de préférence un halogénure d'alkyle renfermant 1 à 6 atomes de carbone par molécule, et de préférence le trichloroéthane ou le tétrachlorure de carbone. La proportion d'halogène ou de composé halogéné employé est telle qu'elle puisse être capable de former 0,2 à 1,2 % en poids d'un dérivé halogéné d'alumine par rapport au catalyseur soumis à la régénération ; l'oxychloration est effectuée à une température comprise entre 350 et 550 °C et sous une pression comprise entre 1 et 15 bar. La durée de ce traitement peut être comprise entre 20 minutes et trois heures. En général, elle est d'environ 1 heure ;
- un troisième traitement correspond à l'oxydation du catalyseur : pour la réaliser, on augmente encore la teneur en oxygène du gaz de régénération admis dans le régénérateur de préférence d'au moins 10 % par rapport à la valeur dans l'étape précédente. La teneur en oxygène est alors le plus souvent comprise entre 3 et 20 % en volume, la température moyenne est comprise encore entre 350 et 550 °C et la pression est comprise entre 1 et 15 bar. La durée de cette opération est comprise, par exemple, entre 30 minutes et deux heures. En général elle est d'environ 1 heure.

Ces trois traitements s'effectuent habituellement soit successivement par exemple dans une enceinte unique à lit fixe, soit dans une enceinte à lit mobile, le catalyseur traversant successivement trois zones distinctes où sont effectuées chacune des étapes de régénération. Ces traitements peuvent également être effectués successivement dans plusieurs enceintes distinctes.

Le catalyseur est alors transvasé du régénérateur vers la première zone catalytique, à travers un système de vannes. En haut de cette zone catalytique le catalyseur est d'abord accumulé dans un volume, distinct de la zone réactionnelle (c'est-à-dire dans lequel ne passe pas le mélange réactionnel contenant des hydrocarbures) où il est réduit, par un courant d'hydrogène, à une température comprise entre 350 et 600 °C et à une pression comprise entre 2 et 25 bar, de préférence entre 4 et 15 bar. La présulfuration du catalyseur peut être éventuellement réalisée dans ce volume. Ensuite le catalyseur frais (et réduit) alimente progressivement, au fur et à mesure du soutirage du catalyseur usé, la zone réactionnelle choisie.

Dans une deuxième variante le fluide de strippage comprendra de l'azote. Selon cette variante du procédé le catalyseur usé ou partiellement usé, provenant de la dernière zone catalytique est dirigé sous azote vers la zone de strippage pour être débarrassé du soufre accumulé dans les zones de réaction. Dans ce cas le strippage a lieu sous atmosphère d'azote à une température comprise entre 400 et 700 °C, de préférence 500 et 600 °C à une pression comprise entre 1 et 4 bar de préférence 1,5 et 2,5 bar et à une VVH comprise entre 100 h⁻¹ et 4 000 h⁻¹ de préférence 700 et 2 500 h⁻¹. Le catalyseur dans la zone de strippage peut être disposé en lit fixe axial ou bien radial. Le strippage peut être effectué en continu ou bien de manière périodique .

Dans cette variante le catalyseur débarrassé du soufre est remonté par un lift à l'azote vers un ballon "accumulateur décanteur" situé par exemple au dessus du régénérateur. Dans le ballon "accumulateur décanteur" l'azote est séparé du catalyseur et dirigé vers un filtre pour éliminer les particules fines de catalyseur et de là envoyé vers un compresseur en vue de son recyclage, par exemple vers les lifts ou la zone de strippage. La régénération s'effectue de manière analogue à la description précédente faite en liaison avec la première variante de réalisation à l'exception de la mise en équilibre de pression du régénérateur avec le ballon "accumulateur décanteur" qui se fait sous atmosphère d'azote. Cette deuxième variante permet d'éliminer la phase de purge à l'azote du régénérateur et/ou du ballon accumulateur dans lequel on a transféré le catalyseur régénéré. Dans cette variante à la fin de la régénération le catalyseur est transféré toujours sous atmosphère d'azote dans un volume, distinct de la zone réactionnelle (c'est-à-dire dans lequel ne passe pas le mélange réactionnel contenant des hydrocarbures) où il est réduit, par un courant d'hydrogène, à une température comprise entre 350 et 600 °C et à une pression comprise entre 2 et 25 bar, de préférence 4 et 15 bar. La présulfuration du catalyseur peut être éventuellement réalisée dans ce volume. Ensuite le catalyseur frais (et réduit) alimente progressivement, au fur et à mesure du soutirage du catalyseur usé, la zone réactionnelle choisie.

La réaction de déshydrogénation s'effectue en général à une pression comprise entre 0,2 et 20 bar absolus (1 bar= 0,1 MPa) et à une température de 400 à 800 °C en fonction de la nature de la charge, la température étant avantageusement de 540 à 700 °C pour le propane et de 500 à 650 °C pour la coupe contenant de l'isobutane sous une pression préférée de 1 à 4 bar absolus. Les vitesses spatiales volumiques (par rapport à la charge liquide) préconisées sont habituellement de 0,5 à 20 h⁻¹ et préférentiellement de 1,5 à 6 h⁻¹. Lorsque la charge comprend de l'hydrogène son taux de recyclage est compris entre 0 et 10 moles d'hydrogène par mole de charge.

Le catalyseur employé dans la présente invention peut être tout catalyseur classique de déshydrogénation bien connu de l'homme de métier. Ce catalyseur est de préférence un catalyseur en grains comprenant sur un support choisi dans le groupe formé par l'alumine et les zéolithes, au moins un métal noble du groupe VIII, au moins un métal du groupe IVA, et au moins un métal du groupe IA ou IIA, de préférence du groupe IA. Les grains du catalyseur ont le plus souvent la forme de billes sensiblement sphériques de diamètre compris généralement entre 1 et 3 millimètres (mm) et de préférence entre 1,5 et 2 mm. La surface spécifique du support est le plus souvent comprise entre 20 et 800 mètres carrés par gramme (m²/g) et de préférence comprise entre 50 et 500 m²/g. Le catalyseur renferme le plus souvent, en poids par rapport au support, 0,01 à 2 % d'au moins un métal noble du groupe VIII, de 0,01 à 3 % d'au moins un métal du groupe IVA et de 0,1 à 3 % d'au moins un métal du groupe IA ou IIA. L'une des formules catalytiques particulièrement préférée renferme, sur un support d'alumine, du platine, de l'étain et du potassium. Le catalyseur peut également renfermer un halogène ou un composé halogéné tel que par exemple du chlore ou un composé de chlore. Il peut aussi renfermer du soufre.

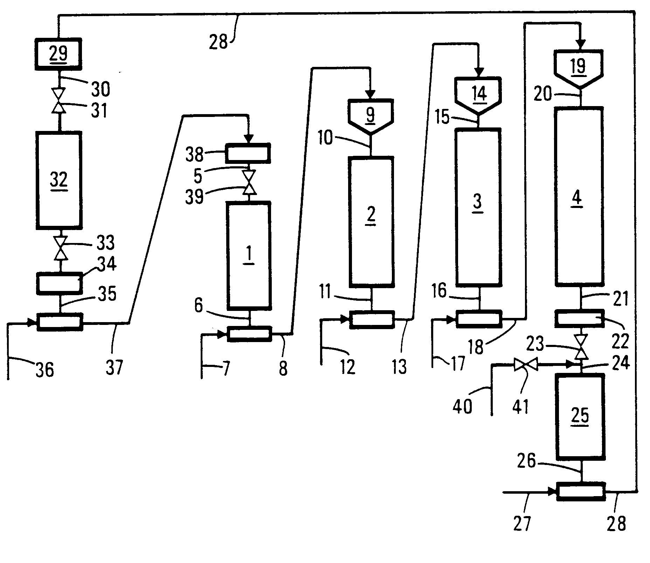

La figure illustre schématiquement l'invention sans toutefois en limiter la portée. Dans la réalisation schématisée sur la figure les zones de réaction sont sensiblement verticales et le catalyseur circule de haut en bas dans chacune d'elle et est transporté par un courant d'hydrogène depuis le bas d'une zone donnée (à l'exception de la dernière) vers le haut de la zone suivante.

Sur la figure, dans un but de simplification, le trajet de la charge circulant successivement à travers les réacteurs en série (1, 2, 3 et 4) n'a pas été illustré.

La charge entre en haut du réacteur (1) et en sort par le bas pour être réintroduite dans le haut du réacteur (2) et ainsi de suite dans les réacteurs (3) et (4). Entre chaque réacteur la charge passe à travers un moyen de chauffage non représenté sur la figure. Le catalyseur, introduit par le conduit (5) en haut du réacteur (1), est soutiré par la conduite (6) puis transféré vers le récipient (9) situé au dessus du réacteur (2) au moyen d'un lift (8) dont le fluide moteur est de l'hydrogène de recyclage, provenant de la section de réaction, introduit par la conduite (7). Le catalyseur contenu dans le récipient (9) est introduit dans le haut du réacteur (2) par la conduite (10). Le catalyseur chemine de la même façon à travers les réacteurs (2), (3) et (4).

Le catalyseur usé, soutiré par la conduite (21) en bas du réacteur (4) est dirigé vers la zone de régénération (32). Dans une première variante le passage d'une atmosphère d'hydrogène à une atmosphère d'azote s'effectue immédiatement en amont du régénérateur au niveau du ballon "accumulateur décanteur" (29), le retour à une atmosphère d'hydrogène s'effectue immédiatement en aval du régénérateur au niveau du ballon accumulateur (34), et l'étanchéité est assurée par les vannes (31) et (33) (en réalité il y a le plus souvent de multiples vannes (31) et (33)), tandis que dans une seconde variante la transition hydrogène/azote a lieu à la sortie du réacteur (4) au niveau du ballon accumulateur (22), la transition azote/hydrogène a lieu immédiatement en amont du premier réacteur (1) au niveau du ballon (38) et l'étanchéité est assurée par les vannes (23) et (39)(en réalité il s'agit le plus souvent d'un système de multiples vannes (23) et (39)). Le catalyseur usé est envoyé par la conduite (24) dans la zone de strippage (25) où il est soumis à un strippage à chaud à l'aide de l'un des gaz de strippage décrit ci-avant (par exemple l'hydrogène chaud ou l'azote chaud) pour le débarrasser d'une partie du soufre accumulé dans les zones de réaction. Le catalyseur, débarrassé du soufre, soutiré par la conduite (26), est ensuite remonté par un lift (28), dont le fluide moteur est dans une première variante de l'hydrogène de recyclage et dans une deuxième variante de l'azote ou un gaz en contenant introduit en partie par la conduite (40) et la vanne (41) et en partie par la conduite (27), vers le ballon "accumulateur décanteur" (29).

Dans ce ballon (29), le fluide moteur après avoir été séparé du catalyseur, est envoyé par une conduite non représentée vers un compresseur de recyclage à travers un système de filtration des particules fines de catalyseur. Le catalyseur usé est ensuite dirigé par la conduite (30) vers le régénérateur (32) qui est mis en équilibre de pression sous atmosphère du gaz moteur employé (le plus souvent hydrogène ou azote) avec le ballon "accumulateur décanteur" (29). Le régénérateur est alors isolé du reste du système par fermeture des vannes (31) et (33), purgé à l'azote, si nécessaire (c'est-à-dire lorsque le gaz moteur n'est pas de l'azote) et la régénération, comprenant les trois traitements décrits ci-avant, y est conduite dans une enceinte unique à lit fixe ou bien dans une enceinte à lit mobile. Lorsque la régénération est effectuée, le régénérateur (32) est si nécessaire purgé à l'azote puis, soit mis sous atmosphère d'hydrogène en équilibre de pression avec le réacteur (1), soit gardé sous atmosphère d'azote ; le catalyseur régénéré est dirigé vers un ballon tampon (34), remonté par la conduite (35) et un lift (37), fonctionnant avec un gaz moteur introduit par la ligne (36) qui est suivant le cas soit de l'hydrogène soit de l'azote dans un récipient accumulateur de catalyseur (38) dans lequel peut être réalisé la réduction et la sulfuration du catalyseur. Le catalyseur régénéré, réduit et sulfuré est alors introduit progressivement dans le réacteur (1). Le catalyseur passe du réacteur (2) au réacteur (3), par la ligne (11), le lift (13) alimenté en hydrogène par la ligne (12), le récipient (14) et la ligne (15). De même il passe du réacteur (3) au réacteur (4), par la ligne (16), le lift (18) alimenté en hydrogène par la ligne (17), le récipient (19) et la ligne (20).

Les exemple suivants, non limitatifs, illustrent l'intérêt de la présente invention sans toutefois en limiter la portée. Ils sont décrits en liaison avec le mode de réalisation schématisé sur la figure.

On a opéré avec 4 réacteurs en série (voir schéma de la figure). La régénération du catalyseur est effectuée sur le catalyseur sortant du quatrième réacteur.

Le catalyseur utilisé dans les exemples est un catalyseur classique supporté sur alumine contenant 0,6 % en poids de platine, 0,9 % en poids d'étain, 0,9 % en poids de potassium et 0.6 % poids de chlore.

La charge d'hydrocarbures que l'on traite a la composition précisée ci-après dans le tableau 1.

**Tableau 1**

| **Charge** | **% poids** |
|---|---|
| Propylène | 0,09 |
| Propane | 1,24 |
| Isobutane | 92,57 |
| Isobutène | 0,02 |
| n-Butane | 6,00 |
| n-Butènes | 0,05 |
| C₅+ | 0,03 |

La charge est additionnée au préalable (c'est-à-dire avant son introduction dans le premier réacteur) de DMDS en une quantité suffisante pour obtenir une charge contenant 50 ppm en poids de soufre. Dans chacun des réacteurs la température d'entrée est de 590 °C et la vitesse volumique spatiale est de 2 volumes de charge liquide (à 15 °C) par volume de catalyseur et par heure. Le taux de recyclage de l'hydrogène est de 1 mole d'hydrogène par mole de charge. La pression à l'entrée du premier réacteur est de 3,2 bar. Les réacteurs sont du type "radial" tels que ceux décrits dans le brevet US-A- 4 277 444.

Le catalyseur est réparti dans les réacteurs dans les rapports pondéraux suivants :
1°) réacteur : 22 %,
2°) réacteur : 24 %,
3°) réacteur : 26 % et
4°) réacteur : 28 %.

La quantité totale de catalyseur est de 22 350 kg. Le catalyseur est renouvelé à un taux de 150 kg/h. Le débit d'H₂ dans les lifts 8, 13, 18, 28 et 37 est de 15 kg/h.

### Exemple 1 (comparatif)

Dans cet exemple le catalyseur usé issu du dernier réacteur ne passe pas par le récipient de strippage (25) et est dirigé directement vers le ballon "accumulateur décanteur" (29) par le lift (28) sous atmosphère d'hydrogène. La régénération est effectuée de la manière suivante :
1) un premier traitement correspond à la combustion du coke, pour laquelle la température à l'entrée de la zone de régénération (32) est maintenue à 420 °C, la pression dans le régénérateur (32) est maintenue à 3,2 bar, la teneur en oxygène du mélange gazeux (azote plus air) introduit est maintenue à 0,5 % en volume et le temps de séjour dans cette zone est de 1 h 30 ;
2) un deuxième traitement oxychlorant, pour laquelle la température à l'entrée de la zone est portée à 510 °C, la pression est maintenue à 3,2 bar, l'injection de trichloroéthane (C₂H₃Cl₃) étant réalisée au débit de 0,8 kg/h, la teneur en oxygène du gaz d'oxychloration à l'entrée de la zone étant de 5 % volume et le temps de séjour du catalyseur est de 1 h ;
3) un troisième traitement correspond à la calcination, pour laquelle la température dans la zone est de 510 °C, la pression est maintenue à 3,2 bar, la teneur en oxygène est de 6 % volume et le temps de séjour du catalyseur est de 1 h.

Le régénérateur (32) est ensuite purgé à l'azote puis mis sous atmosphère d'hydrogène en équilibre de pression avec le premier réacteur.

La réduction du catalyseur est réalisée dans les enceintes (34) et (38) et le lift (37), le débit d'hydrogène étant de 90 kg/h, la température étant maintenue à 530 °C et la pression à 5 bar ; le temps de séjour du catalyseur dans cette enceinte est de 2 heures.

Le catalyseur se désactive rapidement au cours des cycles ce qui se traduit par un empoisonnement irréversible du catalyseur qui entraîne une chute spectaculaire de la conversion, une baisse de la sélectivité en isobutène et par voie de conséquence une chute impressionnante du rendement en isobutène. Les résultats obtenus sont précisés dans le tableau 2 ci-après.

**Tableau 2**

| Heures de marche (h) | Conversion iC₄* = (%poids) | Sélectivité iC₄* = (%poids) | Rendement iC₄* = (%poids) |
|---|---|---|---|
| 250 | 33,5 | 95,2 | 31,9 |
| 1250 | 31,7 | 93,7 | 29,7 |
| 1350 | 21,3 | 92,8 | 19,8 |
| 2300 | 16,3 | 90,7 | 14,8 |
| 2400 | 4,1 | 89,2 | 3,7 |

| | | | |
|---|---|---|---|
| *iC₄= représente l'isobutène | | | |

### Exemple 2 (selon l'invention)

Un deuxième lot de catalyseur de même formulation est préparé. Le même dispositif et les mêmes conditions que celles décrites en liaison avec l'exemple 1 sont utilisés. Le protocole de régénération est identique excepté la réalisation d'une phase de strippage, dans le récipient (25), du catalyseur usé issu du dernier réacteur, avant les traitements de combustion, d'oxychloration et de calcination. Le strippage est effectué, sous hydrogène à une température de 580 °C, avec une vitesse spatiale volumique (par rapport au gaz) de 900 h⁻¹.

Dans ces conditions le catalyseur se révèle très stable au cours des cycles ce qui traduit par un maintien de la conversion à un niveau élevé, une sélectivité en isobutène sensiblement constante au cours des cycles et par voie de conséquence un rendement en isobutène sensiblement constant au cours du temps. Les résultats obtenus sont précisés dans le tableau 3 ci-après.

**Tableau 3**

| Heures de marche (h) | Conversion iC₄* = (%poids) | Sélectivité iC₄* = (%poids) | Rendement iC₄* = (%poids) |
|---|---|---|---|
| 250 | 38,0 | 95,2 | 36,2 |
| 1250 | 37,4 | 94,8 | 35,5 |
| 1350 | 37,1 | 94,1 | 34,9 |
| 2300 | 36,8 | 95,7 | 35,2 |
| 2400 | 37,2 | 94,9 | 35,3 |

| | | | |
|---|---|---|---|
| iC₄* = représente l'isobutène | | | |

## Revendications

1. Procédé continu de déshydrogénation d'hydrocarbures paraffiniques, ayant au moins deux atomes de carbone dans leur molécule, en hydrocarbures oléfiniques, en présence d'un catalyseur, dans lequel on fait circuler une charge contenant lesdits hydrocarbures paraffiniques à travers au moins deux zones de réaction disposées en série, chacune de ces zones de réaction étant de type à lit mobile, la charge circulant successivement à travers chaque zone de réaction et le catalyseur circulant également successivement à travers chaque zone de réaction en s'écoulant en continu ou de façon périodique depuis une première extrémité de ladite zone vers son extrémité opposée, le catalyseur soutiré à partir de l'extrémité de chaque zone de réaction (sauf de la dernière) étant transporté dans un courant de gaz à la première extrémité de la zone de réaction suivante, le catalyseur soutiré de la dernière zone de réaction traversée par la charge étant ensuite envoyé dans une zone de régénération à la sortie de laquelle, le catalyseur régénéré est réintroduit de façon continue ou périodique à proximité de la première extrémité de la première zone de réaction, ledit procédé comprenant une injection d'au moins un élément choisi dans le groupe formé par le soufre et les composés soufrés préalablement ou simultanément à l'introduction de la charge dans la première zone de réaction et étant caractérisé en ce que le catalyseur soutiré de la dernière zone de réaction traversée par la charge est envoyé de façon continue ou périodique dans une zone de strippage dans laquelle il est débarrassé du soufre qu'il renferme, par strippage à l'aide d'un gaz ou d'un mélange de gaz, avant d'être envoyé de façon continue ou périodique dans la zone de régénération.

2. Procédé selon la revendication 1 dans lequel le gaz de strippage comprend au moins un élément choisi dans le groupe formé par l'hydrogène sensiblement pur, l'hydrogène renfermant de 5 à 10 000 ppm en poids d'eau, l'hydrogène renfermant de 5 à 5.000 ppm en poids d'un halogène, l'azote sensiblement pur, l'azote renfermant de 5 à 10 000 ppm en poids d'eau, l'azote renfermant de 5 à 5.000 ppm en poids d'un halogène.

3. Procédé selon la revendication 1 ou 2 dans lequel on injecte dans la charge un composé organique soufré.

4. Procédé selon la revendication 3 dans lequel le composé soufré est choisi dans le groupe formé par les disulfures et les polysulfures organiques, de formule R¹-(S)ₙ-R² dans laquelle R¹ et R², identiques ou différents, représentent chacun un atome d'hydrogène ou un radical hydrocarboné et n est un nombre compris entre 2 et 20.

5. Procédé selon la revendication 3 ou 4 dans lequel le composé soufré est choisi dans le groupe formé par le diméthyldisulfure, le diéthyldisulfure, les dipropyldisulfures, les dibutyldisulfures, le diphényldisulfure, le ditertiododécylpolysulfure (n=5) et le ditertiononylpolysulfure (n=5).

6. Procédé selon l'une des revendications 1 à 5 dans lequel le catalyseur est un catalyseur en grains comprenant, sur un support choisi dans le groupe formé par l'alumine et les zéolithes, au moins un métal noble du groupe VIII, au moins un métal du groupe IVA, et au moins un métal du groupe IA ou du groupe IIA.

7. Procédé selon la revendication 6 dans lequel le catalyseur renferme, en poids par rapport au support, de 0,01 à 2 % d'au moins un métal noble du groupe VIII, de 0,01 à 3 % d'au moins un métal du groupe IVA et de 0,1 à 3 % d'au moins un métal du groupe IA ou du groupe IIA.

8. Procédé selon la revendication 6 ou 7 dans lequel le catalyseur comprend, sur un support d'alumine, du platine, de l'étain et du potassium.

9. Procédé selon l'une des revendications 1 à 8 dans lequel les zones de réaction sont sensiblement verticales et le catalyseur, circulant de haut en bas dans chacune d'elles, est transporté par un courant d'hydrogène depuis le bas d'une zone donnée (à l'exception de la dernière) vers le haut de la zone suivante.

10. Procédé selon l'une des revendications 1 à 9 dans lequel la charge comprend de l'hydrogène.

## Claims

1. A continuous process for the dehydrogenation of paraffinic hydrocarbons having at least two carbon atoms in their molecule into olefinic hydrocarbons in the presence of a catalyst, wherein a charge containing said paraffinic hydrocarbons is circulated through at least two reaction zones arranged in series, each of these reaction zones being of the moving bed type, the charge circulating successively through each reaction zone and the catalyst also circulating successively through each reaction zone flowing continuously or intermittently from a first end of said zone to its opposite end, the catalyst which is drawn off from the end of each reaction zone (except the last) being conveyed in a gas flow to the first end of the following reaction zone, the catalyst which is drawn off from the last reaction zone through which the charge passes then being sent to a regeneration zone at the outlet of which the regenerated catalyst is reintroduced continuously or intermittently close to the first end of the first reaction zone, said process comprising injection of at least one element selected from the group formed by sulphur and compounds of sulphur before or simultaneously to the introduction of the charge into the first reaction zone and being characterised in that the catalyst which is drawn off from the last reaction zone through which the charge passes is sent continuously or intermittently into a stripping zone wherein the sulphur which it contains is removed by stripping by means of a gas or a gas mixture, before being sent continuously or intermittently into the regeneration zone.

2. A process according to Claim 1, wherein the stripping gas comprises at least one element selected from the group formed by substantially pure hydrogen, the hydrogen containing 5 to 10 000 ppm by weight of water, the hydrogen containing 5 to 5.000 ppm by weight of a halogen, substantially pure nitrogen, the nitrogen containing 5 to 10 000 ppm by weight of water, the nitrogen containing 5 to 5.000 ppm by weight of a halogen.

3. A process according to Claim 1 or Claim 2, wherein an organic sulphur compound is injected into the charge.

4. A process according to Claim 3, wherein the sulphur compound is selected from the group formed by organic disulphides and polysulphides of formula R¹-(S)ₙ-R² wherein R¹ and R², which may be the same or different, each represent a hydrogen atom or a hydrocarbon radical and n is a number between 2 and 20.

5. A process according to Claim 3 or Claim 4, wherein the sulphur compound is selected from the group formed by dimethyldisulphide, diethyldisulphide, dipropyldisulphides, dibutyldisulphides, diphenyldisulphide, ditertio-dodecylpolysulphide (n = 5) and ditertiononylpolysulphide (n = 5).

6. A process according to one of Claims 1 to 5, wherein the catalyst is a catalyst composed of grains comprising, on a support selected from the group formed by alumina and zeolites, at least one group VIII noble metal, at least one group IVA metal, and at least one group IA or group IIA metal.

7. A process according to Claim 6, wherein the catalyst contains by weight in relation to the support 0.01 to 2% of at least one group VIII noble metal, from 0.01 to 3% of at least one group IVA metal and from 0.1 to 3% of at least one group IA or group IIA metal.

8. A process according to Claim 6 or Claim 7, wherein the catalyst comprises platinum, tin and potassium on an alumina support.

9. A process according to one of Claims 1 to 8, wherein the reaction zones are substantially vertical and the catalyst which circulates from the top to the bottom in each of these zones is conveyed by a hydrogen current from the bottom of a given zone (with the exception of the last zone) to the top of the following zone.

10. A process according to one of Claims 1 to 9, wherein said charge comprises hydrogen.

## Patentansprüche

1. Kontinuierliches Verfahren zur Dehydrierung paraffinischer Kohlenwasserstoffe mit wenigstens zwei Kohlenstoffatomen in ihrem Molekül zu olefinischen Kohlenwasserstoffen, in Anwesenheit eines Katalysators, bei dem man eine dieser paraffinischen Kohlenwasserstoffe enthaltende Charge durch wenigstens zwei in Reihe angeordnete Reaktionszonen zirkulieren läßt, wobei jede dieser Reaktionszonen vom Typ mit beweglichem Bett ist, die Charge nacheinander durch jede Reaktionszone zirkuliert und der Katalysator ebenfalls nacheinander durch jede Reaktionszone zirkuliert, indem er kontinuierlich oder periodisch von einem ersten Ende der Reaktionszone zu seinem gegenüberliegenden Ende strömt, wobei der aus diesem Ende jeder Reaktionszone (außer der letzteren) abgezogene Katalysator in einem Gasstrom zum ersten Ende der folgenden Reaktionszone transportiert wird, der aus der letzten von der Charge durchsetzten Zone abgezogene Katalysator dann in eine Regenerationszone geschickt wird, an deren Ausgang der regenerierte Katalysator wieder kontinuierlich oder periodisch benachbart dem ersten Ende der ersten Reaktionszone eingeführt wird, das Verfahren eine Injektion von wenigstens einem Element umfaßt, das aus der Gruppe gewählt ist, die durch Schwefel und die schwefelhaltigen Verbindungen gebildet ist, und zwar vor oder gleichzeitig zu dem Einführen der Charge in die Reaktionszone, wobei das Verfahren sich dadurch auszeichnet, daß der aus der letzten von der Charge durchsetzten Reaktionszone abgezogene Katalysator kontinuierlich oder periodisch in eine Stripperzone eingeführt wird, in der er von dem Schwefel, den er umfaßt, durch Strippen vermittels eines Gases oder eines Gasgemisches befreit wird, bevor er kontinuierlich oder periodisch in die Regenerationszone geschickt wird.

2. Verfahren nach Anspruch 1, bei dem das Strippergas wenigstens ein Element umfaßt, das ausgewählt ist aus der Gruppe, die aus im wesentlichen reinem Wasserstoff, Wasserstoff, der 5 bis 10000 ppm Wasser enthält, Wasserstoff, der 5 bis 5000 ppm eines Halogens enthält, im wesentlichen reinem Stickstoff, Stickstoff, der 5 bis 10000 ppm Wasser enthält, Stickstoff, der 5 bis 5000 Gewichtsppm eines Halogens enthält, gebildet wird.

3. Verfahren nach Anspruch 1 oder 2, bei dem man in die Charge eine schwefelhaltige organische Verbindung injiziert.

4. Verfahren nach Anspruch 3, bei dem die schwefelhaltige Verbindung gewählt ist aus der Gruppe, die gebildet wird durch die Disulfide und die organischen Polysulfide mit der Formel R¹-(S)ₙ-R², in welcher R¹ und R², identisch oder unterschiedlich, je ein Wasserstoffatom oder ein kohlenwasserstoffhaltiges Radikal darstellen und n eine Zahl zwischen 2 und 20 ist.

5. Verfahren nach Anspruch 3 oder 4, bei dem die schwefelhaltige Verbindung gewählt ist aus der Gruppe, die gebildet wird durch das Dimethyldisulfid, das Diethyldisulfid, die Dipropyldisulfide, die Dibutyldisulfide, das Diphenyldisulfid, das Di-tert-Dodecylpolysufid (n=5) und das Di-tert-nonylpolysulfid (n=5).

6. Verfahren nach einem der Ansprüche 1 bis 5, bei dem der Katalysator ein Katalysator in Körnerform ist, der auf einem Träger, gewählt aus der durch Aluminiumoxid und Zeolithe gebildeten Gruppe, wenigstens ein Edelmetall der Gruppe VIII, wenigstens ein Metall der Gruppe IVA und wenigstens ein Metall der Gruppe IA oder der Gruppe IIA umfaßt.

7. Verfahren nach Anspruch 6, bei dem der Katalysator in Gewicht, bezogen auf den Träger, zwischen 0,01 bis 2 % wenigstens eines Edelmetalls der Gruppe VIII, zwischen 0,01 bis 3 % wenigstens eines Metalls der Gruppe IVA und von 0,3 bis 1 % wenigstens eines Metalls der Gruppe IA oder der Gruppe IIA umfaßt.

8. Verfahren nach Anspruch 6 oder 7, bei dem der Katalysator auf einem Aluminiumoxidträger Platin, Zinn und Kalium umfaßt.

9. Verfahren nach einem der Ansprüche 1 bis 8, bei dem die Reaktionszonen im wesentlichen vertikal sind und der Katalysator, der von oben nach unten in jeder hiervon zirkuliert, durch einen Wasserstoffstrom von unten aus einer gegebenen Zone (mit Ausnahme der letzteren) nach oben zur folgenden Zone transportiert wird.

10. Verfahren nach einem der Ansprüche 1 bis 9, bei dem die Charge Wasserstoff umfaßt.
